(19) Europäisches Patentamt
European Patent Office
Office européen des brevets

(11) Numéro de publication : **0 607 077 A1**

(12) **DEMANDE DE BREVET EUROPEEN**

(21) Numéro de dépôt : **94400058.7**

(22) Date de dépôt : **11.01.94**

(51) Int. Cl.$^5$ : **C07D 403/10,** C07D 239/36, A61K 31/505

(30) Priorité : **15.01.93 FR 9300340**

(43) Date de publication de la demande : **20.07.94 Bulletin 94/29**

(84) Etats contractants désignés : **AT BE CH DE DK ES FR GB GR IE IT LI LU MC NL PT SE**

(71) Demandeur : **SYNTHELABO**
**22, Avenue Galilée**
**F-92350 Le Plessis Robinson (FR)**

(72) Inventeur : **Hoornaert, Christian**
**58 avenue de Choisy**
**F-75013 Paris (FR)**
Inventeur : **Wick, Alexander-Eduard**
**10 boulevard des Plants**
**F-78860 St-Nom-la-Breteche (FR)**

(74) Mandataire : **Thouret-Lemaitre, Elisabeth et al**
**SYNTHELABO,**
**Service Brevets,**
**B.P. 72**
**F-92352 Le Plessis-Robinson Cédex (FR)**

(54) **Sels de dérivés de 4-pyrimidinone, leur préparation et leur application en thérapeutique.**

(57) Sels d'alcanolamines et d'acides aminés basiques de dérivés pouvant exister sous la forme de tautomères de formule (I), (I') et (I")

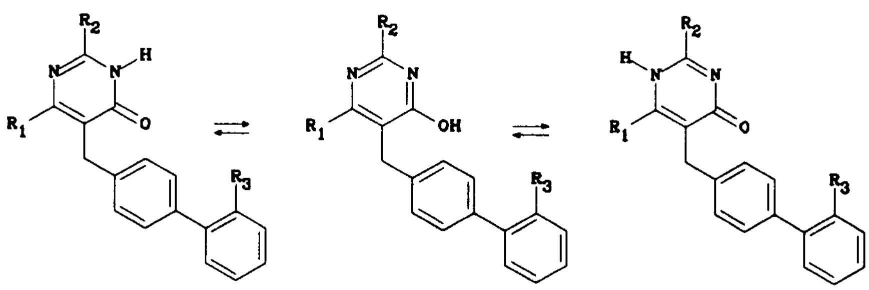

(I) (I') (I")

dans laquelle
$R_1$ représente un groupe $(C_1-C_7)$alkyle droit ou ramifié,
$R_2$ représente soit un groupe aryl$(C_1-C_3)$alkyle éventuellement substitué sur le noyau, soit un groupe hétéroaryl$(C_1-C_3)$ alkyle éventuellement substitué sur le noyau et
$R_3$ représente soit un groupe COOH, soit un groupe 1*H*-tétrazol-5-yle.
Application en thérapeutique.

EP 0 607 077 A1

Jouve, 18, rue Saint-Denis, 75001 PARIS

La présente invention a pour objet des sels organiques pharmacologiquement acceptables de dérivés de 4-pyrimidinone pouvant exister sous trois formes tautomères répondant aux formules (I), (I') et (I")

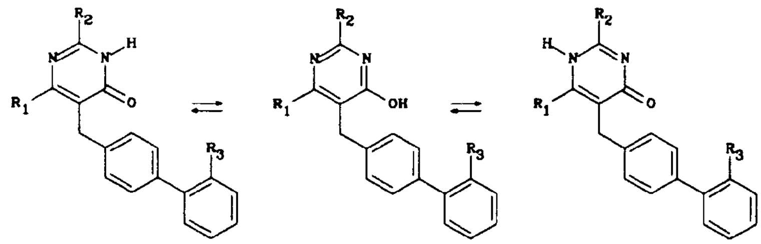

(I) (I') (I")

dans laquelle

$R_1$ représente un groupe $(C_1\text{-}C_7)$alkyle droit ou ramifié,

$R_2$ représente soit un groupe aryl$(C_1\text{-}C_3)$alkyle éventuellement substitué sur le noyau, soit un groupe hétéroaryl$(C_1\text{-}C_3)$alkyle éventuellement substitué sur le noyau et

$R_3$ représente soit un groupe COOH, soit un groupe 1*H*-tétrazol-5-yle,

leur préparation et leur application en thérapeutique.

Les sels des formes tautomères (I') et (I") font aussi partie de l'invention.

Parmi les sels selon l'invention, les sels préférés sont les sels des dérivés de formule (I) dans laquelle

$R_1$ représente un groupe butyle,

$R_2$ représente soit un groupe phényléthyle, soit un groupe 4-méthoxyphényléthyle, soit un groupe 3-fluoro-4-méthoxyphényléthyle, soit un groupe 3,4,5-triméthoxyphényléthyle, soit un groupe 3-pyridinyléthyle, soit un groupe 4-pyridinyléthyle et

$R_3$ représente un groupe 1*H*-tétrazol-5-yle.

Parmi ceux-ci, les sels de choix sont les sels de la 6-butyl-2-(2-phényléthyl)-5-[[2'-(1*H*-tétrazol-5-yl)[1,1'-biphényl]-4-yl]méthyl]pyrimidin-4(1*H*)-one.

Les différents sels selon l'invention peuvent être préparés par des méthodes connues de l'homme du métier.

Ainsi on peut salifier les composés de formule (I) par exemple par différentes bases organiques, telles que les alcanolamines comme le 2-aminoéthanol, le 2,2'-iminobiséthanol, le 2,2',2"-nitrilotriéthanol, le tris(hydroxyméthyl)aminométhane ou la N-méthylglucamine ou par des acides aminés basiques comme la lysine ou l'arginine sous forme racémique ou optiquement pure.

Les différentes bases organiques et acides aminés basiques sont disponibles dans le commerce.

Les composés de formule (I) et leurs formes tautomères sont décrits dans la demande de brevet européen no 0500409 de la demanderesse.

La salification des composés de formule (I) par des alcanolamines et par des acides aminés basiques augmente de manière surprenante leur solubilité dans l'eau tamponnée à pH 6,8 et améliore, également de manière surprenante, leur absorption lors de leur administration par voie orale.

Les exemples suivants illustrent l'invention.

Les microanalyses et les spectres IR et RMN confirment la structure des composés obtenus.

## <u>Exemple 1</u>

Sel de 2-aminoéthanol de la 6-butyl-2-(2-phényléthyl)-5-[[2'-(1*H*-tétrazol-5-yl)[1,1'-biphényl]-4-yl]méthyl]pyrimidin-4 (1*H*)-one

A une suspension de 1,47 g (3 mmoles) de 6-butyl-2-(2-phényléthyl)-5-[[2'-(1*H*-tétrazol-5-yl)[1,1'-biphényl]-4-yl]méthyl]pyrimidin-4(1*H*)-one dans 10 ml de méthanol, on ajoute 0,191 g (3,12 mmoles) de 2-aminoéthanol en solution dans 5 ml de méthanol. On laisse sous agitation à la température ambiante pendant quelques minutes, on filtre sur coton et on évapore sous vide. On dissout la mousse obtenue dans 25 ml de dichlorométhane et on ajoute progressivement, sous bonne agitation, 20 ml de diisopropyléther. On laisse le mélange réactionnel une nuit à la température ambiante. On filtre le précipité formé, on le rince avec 10 ml de diisopropyléther et on le sèche sous vide, à 50 °C, en présence de pentoxyde de phosphore.

On obtient 1,6 g de produit.

Point de fusion = 127-130 °C

Exemple 2

Sel de 2,2'-iminobiséthanol de la 6-butyl-2-(2-phényléthyl)-5-[[2'-(1*H*-tétrazol-5-yl)[1,1'-biphényl]-4-yl]méthyl]pyrimidin-4(1*H*)-one

A 1,96 g (4 mmoles) de 6-butyl-2-(2-phényléthyl)-5-[[2'-(1*H*-tétrazol-5-yl)[1,1'-biphényl]-4-yl]méthyl]pyrimidin-4(1*H*)-one, on ajoute 0,42 g (4 mmoles) de 2,2'-iminobiséthanol en solution dans 30 ml de méthanol. On laisse sous agitation à la température ambiante pendant quelques minutes, on filtre sur papier, on rince par 10 ml de méthanol et on évapore sous vide. On redissout à chaud la mousse résiduelle dans 20 ml d'isopropanol puis on ajoute progressivement, sous bonne agitation, 20 ml de diisopropyléther. On filtre le précipité blanc formé, on le rince par 20 ml de diisopropyléther et on le sèche sous vide, à 55 °C, en présence de pentoxyde de phosphore.
On obtient 2,15 g de produit.
Point de fusion = 124-126 °C

Exemple 3

Sel de *L*-(+)-lysine de la 6-butyl-2-(2-phényléthyl)-5-[[2'-(1*H*-tétrazol-5-yl)[1,1'-biphényl]-4-yl]méthyl]pyrimidin-4 (1*H*)-one

A 0,962 g (1,96 mmoles) de 6-butyl-2-(2-phényléthyl)-5-[[2'-(1*H*-tétrazol-5-yl)[1,1'-biphényl]-4-yl]méthyl]pyrimidin-4 (1*H*)-one, on ajoute 0,287 g (1,96 mmoles) de *L*-(+)-lysine en solution dans 15 ml de méthanol. On laisse sous agitation à la température ambiante pendant quelques minutes, on filtre, sur coton, on rince par 5 ml de méthanol et on évapore sous vide. On redissout à chaud la poudre blanche résiduelle dans 15 ml de dichlorométhane et on ajoute progressivement, sous bonne agitation, 30 ml de diisopropyléther. On laisse à la température ambiante pendant 3 jours puis on filtre le précipité formé. On évapore les eaux-mères, on redissout la poudre blanche résiduelle dans 5 ml de dichlorométhane et on ajoute progressivement, sous bonne agitation, 10 ml de diisopropyléther. On filtre le précipité formé. On rassemble les deux précipités, on les sèche sous vide, à 50 °C, en présence de pentoxyde de phosphore.
On obtient 0,7 g de produit.
Point de fusion = 80-120 °C (décomposition)
$[\alpha]_D^{20}$ = + 5,1 (c = 1%, méthanol)

Exemple 4

Sel de *L*-(+)-arginine de la 6-butyl-2-(2-phényléthyl)-5-[[2'-(1*H*-tétrazol-5-yl)[1,1'-biphényl]-4-yl]méthyl]pyrimidin-4 (1*H*)-one

On agite à la température ambiante, jusqu'à dissolution, une suspension de 3,63 g (7,4 mmoles) de 6-butyl-2-(2-phényl-é- thyl)-5-[[2'-(1*H*-tétrazol-5-yl)[1,1'-biphényl]-4-yl]méthyl]pyrimidin-4(1*H*)-one et de 1,29 g (7,4 mmoles) de *L*-(+)-arginine dans 75 ml de méthanol. On concentre sous vide, on reprend la mousse résiduelle dans 80 ml de dichlorométhane chaud, on filtre sur papier et on rince par 10 ml de dichlorométhane chaud. On ajoute progressivement, sous bonne agitation, 120 ml de diisopropyléther. On laisse à la température ambiante pendant 3 jours puis on filtre le précipité blanc obtenu et on le sèche sous vide, à 50 °C, en présence de pentoxyde de phosphore.
On obtient 1,2 g de produit.
Point de fusion = 120-200 °C (décomposition)
$[\alpha]_D^{20}$ = + 6,1 (c = 1%, méthanol)

Exemple 5

Sel de *DL*-lysine de la 6-butyl-2-(2-phényléthyl)-5-[[2'-(1*H*-tétrazol-5-yl)[1,1'-biphényl]-4-yl]méthyl]pyrimidin-4(1*H*)-one On agite jusqu'à dissolution, en chauffant, une suspension de 0,980 g (2 mmoles) de 6-butyl-2-(2-phényléthyl)-5-[[2'-(1*H*-tétrazol-5-yl)[1,1'-biphényl]-4-yl]méthyl)pyrimidin-4(1*H*)-one et de 0,292 g (2 mmoles) de DL-lysine dans 10 ml de méthanol. On filtre sur papier et on rince par 5 ml de méthanol chaud. On ajoute ensuite progressivement, sous bonne agitation, 17 ml de diisopropyléther. On laisse à la température ambiante pendant une nuit puis on filtre le précipité blanc obtenu. On le rince successivement par 10 ml d'un mélange méthanol: diisopropyléther (1:1) puis par 10 ml de diisopropyléther et on le sèche sous vide, à 60 °C, en présence de pentoxyde de phosphore.
On obtient 0,95 g de produit.

Point de fusion = 200-204 °C (décomposition)

Les composés de l'invention ont fait l'objet d'études pharmacologiques qui ont mis en évidence leurs propriétés antagonistes de l'angiotensine II. Ils ont été testés chez le rat spontanément hypertendu traité à l'hydrochlorothiazide.

On utilise des rats mâles (SHR, Charles River France) pesant 400 à 450 g que l'on maintient en contention dans une enceinte thermostatée à 28 °C (degré d'hygrométrie = 70 à 80 %). On place deux manchons à la base de la queue : un capteur piézo-électrique permettant l'enregistrement du pouls caudal (et de la fréquence cardiaque par intégration du signal) et un manchon pneumatique de gonflage permettant l'arrêt du flux sanguin lorsque la contre-pression est égale à la pression artérielle systolique de l'animal.

On entraîne au préalable les animaux à la mesure de la pression artérielle par cette méthode.

On prétraite les animaux par voie orale en injectant une dose d'hydrochlorothiazide de 6 mg/kg afin d'augmenter le taux d'angiotensine II circulante; 30 minutes après le prétraitement, on administre les produits à tester en suspension dans une solution de Tween à 0,2 %, par voie orale. On place les animaux dans l'enceinte thermostatée et on mesure la pression artérielle (et la fréquence cardiaque) aux temps 1 heure, 3 heures, 7 heures, 24 heures et 48 heures après le traitement.

Le pourcentage de baisse de la pression artérielle est utilisé pour apprécier le potentiel antagoniste à l'angiotensine II des sels de l'invention.

Les sels de l'invention peuvent être utilisés pour le traitement de diverses formes de pathologies hypertensives et d'insuffisances coronaire, cardiaque, rénale ou pulmonaire ainsi que pour le traitement du glaucome.

Les sels de l'invention peuvent également être utilisés en association avec d'autres substances à activité cardio-vasculaire, telles que les diurétiques, les $\alpha$-bloquants, les $\beta$-bloquants, les antagonistes calciques ou les inhibiteurs de l'enzyme de conversion de l'angiotensine I.

Les sels de l'invention peuvent être présentés sous toutes formes pharmaceutiques appropriées au traitement pour l'administration orale, parentérale, intramusculaire ou rectale : comprimés, capsules, gélules, solutions ou suspensions stériles, suppositoires etc...

Pour le traitement du glaucome, les sels de l'invention peuvent être présentés sous la forme de comprimés, gélules, solutions injectables ou formulations oculaires topiques.

Les sels de l'invention peuvent être administrés aux patients en une quantité pouvant aller de 1 à 1000 mg par jour et par patient, en une ou plusieurs doses.

## Revendications

1. Sels d'alcanolamines et sels d'acides aminés basiques de dérivés pouvant exister sous trois formes tautomères de formules (I), (I') et (I")

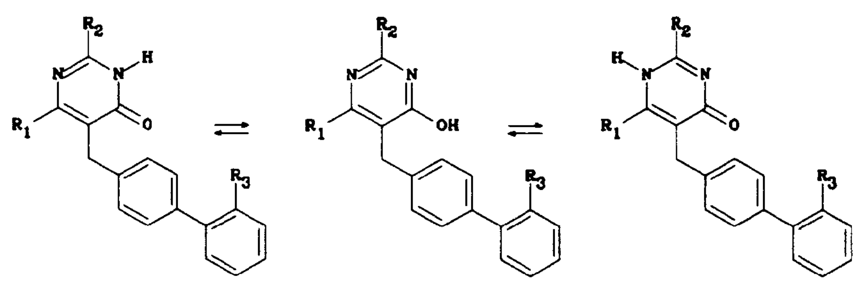

(I) (I') (I")

dans laquelle

$R_1$ représente un groupe ($C_1$-$C_7$)alkyle droit ou ramifié,

$R_2$ représente soit un groupe aryl($C_1$-$C_3$)alkyle éventuellement substitué sur le noyau, soit un groupe hétéroaryl($C_1$-$C_3$) alkyle éventuellement substitué sur le noyau et

$R_3$ représente soit un groupe COOH, soit un groupe 1*H*-tétrazol-5-yle.

2. Sels selon la revendication 1, caractérisés en ce que le dérivé répond à la formule (I) dans laquelle

$R_1$ représente un groupe butyle,

$R_2$ représente soit un groupe phényléthyle, soit un groupe 4-méthoxyphényléthyle, soit un groupe 3-fluoro-4-méthoxyphényléthyle, soit un groupe 3,4,5-triméthoxyphényléthyle, soit un groupe 3-pyridinyléthyle, soit un groupe 4-pyridinyléthyle et
$R_3$ représente un groupe 1*H*-tétrazol-5-yle.

**3.** Sels d'alcanolamines et d'acides aminés basiques de la 6-butyl-2-(2-phényléthyl)-5-[[2'-(1*H*-tétrazol-5-yl)[1,1'-biphényl]-4-yl]méthyl]pyrimidin-4(1*H*)-one.

**4.** Sel de *DL*-lysine de la 6-butyl-2-(2-phényléthyl)-5-[[2'-(1*H*-tétrazol-5-yl)[1,1'-biphényl)-4-yl)méthyl)pyrimidin-4(1*H*)-one.

**5.** Médicament, caractérisé en ce qu'il contient un composé selon l'une quelconque des revendications 1 à 4.

**6.** Composition pharmaceutique, caractérisée en ce qu'elle contient un composé selon l'une quelconque des revendications 1 à 4, associé à tout excipient pharmaceutiquement acceptable.

Office européen des brevets

# RAPPORT DE RECHERCHE EUROPEENNE

Numero de la demande
EP 94 40 0058

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | EP-A-0 500 409 (SYNTHELABO)<br>* page 5 - page 17; exemples 2-10 *<br>----- | 1,2,5,6 | C07D403/10<br>C07D239/36<br>A61K31/505 |
| | | | **DOMAINES TECHNIQUES RECHERCHES (Int.Cl.5)** |
| | | | C07D |

Le présent rapport a été établi pour toutes les revendications

| Lieu de la recherche | Date d'achèvement de la recherche | Examinateur |
|---|---|---|
| LA HAYE | 8 Avril 1994 | Francois, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)